(19) Europäisches Patentamt
European Patent Office
Office européen des brevets

(11) **EP 2 025 354 A1**

(12) **EUROPEAN PATENT APPLICATION**
published in accordance with Art. 153(4) EPC

(43) Date of publication:
**18.02.2009 Bulletin 2009/08**

(21) Application number: **07743205.2**

(22) Date of filing: **11.05.2007**

(51) Int Cl.:
***A61L 31/00** (2006.01)* ***A61K 31/785** (2006.01)*
***A61P 7/04** (2006.01)* ***A61P 41/00** (2006.01)*

(86) International application number:
**PCT/JP2007/059770**

(87) International publication number:
**WO 2007/132785 (22.11.2007 Gazette 2007/47)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **16.05.2006 JP 2006136991**

(71) Applicants:
- **Keio University**
  **Tokyo 108-8345 (JP)**
- **Meiji Seika Kaisha Ltd.**
  **Tokyo 104-8002 (JP)**

(72) Inventors:
- **IZUMI, Yotaro**
  **Shinjuku-ku, Tokyo 1608582 (JP)**
- **KAWAMURA, Masafumi**
  **Shinjuku-ku, Tokyo 1608582 (JP)**
- **KOBAYASHI, Koichi**
  **Shinjuku-ku, Tokyo 1608582 (JP)**
- **ENDO, Takeshi**
  **Iizuka-shi, Fukuoka 8208555 (JP)**
- **NAGAI, Atsushi**
  **Kanagawa-ku, Kanagawa 2218686 (JP)**
- **SHICHINOHE, Makoto**
  **Chuo-ku, Tokyo 1048002 (JP)**

(74) Representative: **Ahner, Francis et al**
**Cabinet Régimbeau,**
**20, rue de Chazelles**
**75847 Paris Cedex 17 (FR)**

(54) **AGENT FOR PREVENTING ORGAN ADHESION AND METHOD FOR PREVENTING ADHESION USING THE SAME**

(57) There is disclosed an organ-adhesion preventing agent comprising a chemically-crosslinked γ-polyglutamic acid as an effective ingredient, as well as a process for preventing organ from adhesion comprising a step of bringing the adhesion-preventing agent into contact with or depositing the same onto the surface of a local organ and optionally the neighborhood thereof to be prevented from the post-operative adhesion. According to the present invention, there is provided an adhesion-preventing agent which is capable of effectively preventing the surfaces of organs from adhesion, and which can be absorbed in the body with high safety and be produced with ease and has high practical usefulness, as well as a process for preventing organ from adhesion by using the adhesion-preventing agent.

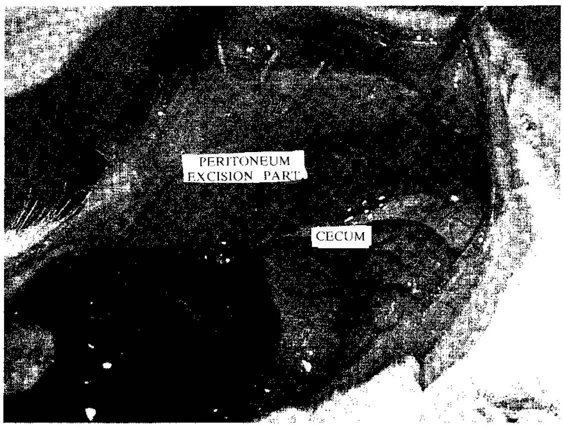


F I G. 2

EP 2 025 354 A1

## Description

### RELATED APPLICATIONS

The present application claims a priority of Japanese Patent Application No. 2006-136991 filed on May 16, 2006, the disclosure of which is incorporated herein by reference.

### BACKGROUND OF THE INVENTION

### Technical Field

The present invention relates to an organ-adhesion preventing agent and a process for preventing adhesion using thereof, in particular to an organ-adhesion preventing agent which is capable of effectively preventing the surfaces of organs from adhesion and which can be absorbed in the body with high safety and manufactured with ease, as well as to a process for preventing adhesion by using the adhesion-preventing agent.

### Background Art

Organs are generally in a state which can be freely moved or separated with each other even if the surface of an organ is in proximal contact with that of the other organ. However, adhesion between organs may be caused by inflammation due to operation or some other causes, which brings about deuteropathy such as hypofunctions of local organs. When the adhesion of organs is caused, it is necessary to conduct re-operation, which increases the burden on the patient, and the operation is very difficult to be completed.
Adhesion-preventing agents have been proposed for the purpose of preventing the adhesion of organs.
Adhesion-preventing agents are broadly classified into non-absorbent and absorbent materials. The non-absorbent materials include silicone sheet, GoreTex™ sheet, and organ-adhesion preventing films comprising a hydrogel film of a mixed polymer which comprises polyvinyl alcohol and a prescribed water-soluble polymer (Japanese Patent Laid-Open Publication No. 266615/1996, which is incorporated herein by reference). While these sheets physically isolate a damaged site by affixing the sheet to the site, resulting in no adhesion, they are not absorbed in the body and thus offer a problem that an artificial material remains in the body.
Furthermore, the non-absorbent sheet or film thus affixed itself may cause dysfunction by adhering to organs. In this case, it will be impossible to remove the sheet thus adhered to the organs even by re-operation.
Therefore, it has been attempted to develop absorbent base materials which can be absorbed in the body with high safety.
There have been proposed, as the absorbent base material, gelatin, fibrin glue, hyaluronic acid and salts thereof, cellulose, as well as an adhesion-preventing agent comprising poly-(y-glutamate) complexes formed by hydrogen bonding of the carboxy anion of the poly-(γ-glutamate) with chitosan or the like (Japanese Patent Laid-Open Publication No. 76572/1999, which is incorporated herein by reference), a radiation-sterilizable medical material comprising a bioavailable polymer and a multi-functional triazine compound such as triallyl-isocyanurate contained therein (Japanese Patent Laid-Open Publication No. 695/2003, which is incorporated herein by reference), and the like.
Moreover, there have been proposed, as a crosslinked poly-(y-glutamate) polymer obtained by crosslinking the poly-(y-glutamic acid) as a single polymer, a γ-radiation-crosslinked poly-(y-glutamic acid) (Japanese Patent Laid-Open Publication No. 322358/1994, which is incorporated herein by reference), a polymeric gel of the poly-(γ-glutamic acid) obtained by chemical reactions (Japanese Patent Laid-Open Publication No. 256220/1994, which is incorporated herein by reference), a grafted poly-(γ-glutamic acid) obtained by the chemical reaction of poly-(γ-glutamic acid) with polypropylene glycol or polyethylene imine (Japanese Patent Laid-Open Publication No. 298533/1992, which is incorporated herein by reference), and the like. Furthermore, there are disclosed crosslinked poly-(γ-glutamic acids) also in Japanese Patent Laid-Open Publication Nos. 343339/1999 and 128899/2002, all of which are fully incorporated herein by reference. However, no applications as a post-operative adhesion-preventing agent are disclosed in these references.
Some of the present inventors have previously filed a patent application on an adhesion preventive comprising a water-absorbent crosslinked poly-γ-glutamic acid (see PCT/JP2005/021094, which is incorporated herein by reference).

### SUMMARY OF THE INVENTION

The present inventors have now found that a chemically-crosslinked poly-(γ-glutamic acid) (referred to hereinafter as γ-polyglutamic acid) is effective for preventing the adhesion of organs. The present invention is based on such findings.
Thus, An object of the present invention is to provide an organ-adhesion preventing agent (material) and a

process for preventing adhesion with use of the adhesion-preventing agent.

According to an aspect of the present invention, there is provided an organ-adhesion preventing agent comprising a chemically-crosslinked γ-polyglutamic acid as an effective ingredient.

According to another aspect of the present invention, there is provided a process for preventing organ from adhesion, comprising a step of: bringing the adhesion-preventing agent into contact with or depositing the same onto the surface of a local organ and optionally the neighborhood thereof to be prevented from the post-operative adhesion.

According to an aspect of the present invention, the use of the chemically-crosslinked γ-polyglutamic acid is advantageous in that it is manufactured with ease and has a viscosity in such range that it will not be washed off by circumferential moisture, as well as that it is capable of efficiently absorbing exudate from the surface of wound. In addition, the chemically-crosslinked γ-polyglutamic acid is also advantageous in that it is absorbed in the body without side effects and with high safety. Furthermore, according to an aspect of the present invention, is also advantageous in that it is capable of not only effectively preventing the adhesion at the surface of post-operative local organs, but also stopping bleeding, which is of a small amount.

## BRIEF DESCRIPTION OF DRAWINGS

Fig. 1 shows the state of adhesion in the group non-treated with the chemically-crosslinked γ-polyglutamic acid in Example 1.

Fig. 2 shows the state of the prevention of adhesion in the group treated with the chemically-crosslinked γ-polyglutamic acid in Example 1.

## DETAILED DESCRIPTION OF THE INVENTION

As used herein, the term "chemically-crosslinked γ-polyglutamic acid" means a water absorbent polymer which comprises a biodegradable γ-polyglutamic acid having a carboxyl side chain, the γ-polyglutamic acid molecules being crosslinked to each other by the chemical reaction with a compound which has two or more functional groups reacting with the carboxyl group in the same molecule, i.e., crosslinking agent.

The chemically-crosslinked γ-polyglutamic acid is the γ-polyglutamic acid as a hydrophilic polymer molecule, which is in the form of a matrix structure by crosslinking formation and has the capacity of maintaining water in the structure. According to the preferred embodiment of the present invention, the chemically-crosslinked γ-polyglutamic acid is preferably in a gel state, but even if the solution after crosslinking reaction is not gelled, the chemically-crosslinked γ-polyglutamic acid can be used in the present invention as far as it has an effect of preventing organ from adhesion. The water absorption property of the chemically-crosslinked γ-polyglutamic acid is so favorable as to retain water in several hundred times of its weight.

The crosslinking of the γ-polyglutamic acid means, for example, the crosslinking of one γ-polyglutamic acid molecule with the other γ-polyglutamic acid molecule directly or through a crosslinking agent.

As used herein, the term "γ-polyglutamic acid" means a polymer of glutamic acid, in which D- and L-glutamic acids are linked in the γ-position to form an acidic water soluble polymer having a molecular weight preferably in the range of 200,000 - 2,000,000. The γ-polyglutamic acid may be in the form of salt, which includes, for example, alkali metal and alkaline earth metal salts. Also, the γ-polyglutamic acid may be those composed only of the D-glutamic acid or those composed only of the L-glutamic acid.

The γ-polyglutamic acid is a main ingredient of a viscous substance in fermented soybeans and commonly used for food, confirming safety for organisms. In addition, it is biodegraded in soil and thus of no risk of environmental pollution. Therefore, it has been recognized useful for moisture retention of the skin and currently used for cosmetics.

The chemically-crosslinked γ-polyglutamic acid may be prepared by crosslinking the γ-polyglutamic acids by chemical reaction of the γ-polyglutamic acid and a crosslinking agent, optionally in the presence of a condensation agent. The reaction can be conducted by appropriately selecting the conditions depending on the amount and kind of the crosslinking agent and a condensation agent to be used if necessary.

The chemically-crosslinked γ-polyglutamic acid can be specifically manufactured by the methods described in Japanese Patent Laid-Open Publication Nos. 298533/1992, 256220/1994, 343339/1999 and 128899/2002. That is to say, the γ-polyglutamic acid or a salt thereof as the raw material can be directly reacted with a compound having two or more functional groups which can be reacted with the carboxyl group at the side chain of the γ-polyglutamic acid in the single molecule (crosslinking agent) in a solvent, or reacted with a crosslinking agent in the presence of a condensation agent in a solvent, to generate the chemically-crosslinked γ-polyglutamic acid.

The non-crosslinked γ-polyglutamic acid is not particularly limited and may be the one produced by the microbes Bacillus genus including Bacillus subtilis, Bacillus anthracis, Bacillus megaterium or Bacillus natto (see Biosci. Biotech.,

56, 1031-1035 (1992) and Japanese Patent Laid-Open Publication No. 174397/1989, all of which are incorporated herein by reference), or may be the one obtained by chemical synthesis. Also, the process for purifying the γ-polyglutamic acid obtained is not particularly limited and may be carried out according to the process described in Japanese Patent Laid-Open Publication No. 316286/1995, which is incorporated herein by reference.

The crosslinking agent used for producing the chemically-crosslinked γ-polyglutamic acid used in the present invention is not particularly limited as far as the chemically-crosslinked γ-polyglutamic acid can be obtained by the reaction of the carboxyl group in the glutamic acid as the building unit of the γ-polyglutamic acid (also referred to hereinafter as the monomer unit) and the functional groups of the crosslinking agent, and includes, for example, polyepoxy compound, polyol, polyamine or polyisocyanate as well as diol, diamine and diisocyanate. In this connection, the aforementioned monomer unit is represented by the following formula.

[Formula 1] $-CO-CH_2-CH_2-CH(COOH)-NH-$

According to the preferred embodiment of the present invention, the crosslinked γ-polyglutamic acid having the desired property can be obtained by controlling the ratio of the total number of the carboxyl groups in the glutamic acid as the monomer unit and the number of the crosslinking agent molecule.

According to the preferred embodiment of the present invention, the chemically-crosslinked γ-polyglutamic acid is preferably produced by using the crosslinking agent and the γ-polyglutamic acid in a ratio in the range of 1:100 - 1:2, the ratio being of the total number of moles of the crosslinking agent and the total number of moles of the glutamic acid (monomer unit) as the building unit of the γ-polyglutamic acid. The crosslinking reaction proceeds sufficiently with the amount of the crosslinking agent in the range described above, and thereby high water absorption coefficient can be guaranteed. According to the preferred embodiment of the present invention, the aforementioned ratio is more preferably in the range of 1:40 - 3:8, and further preferably in the range of 1:35 - 1:10.

The polyepoxy compound as the crosslinking agent specifically includes (poly)ethylene glycol diglycidyl ether (the number of the ethylene glycol unit in the molecule being preferably in the range of 1 - 6, more preferably in the range of 1 - 4), (poly)propylene glycol diglycidyl ether (the number of the propylene glycol unit in the molecule being preferably in the range of 1 - 6, more preferably in the range of 1 - 4), glycerin diglycidyl ether (preferably glycerin-1,3-diglycidyl ether), and the like. According to the preferred embodiment of the present invention, the (poly)ethylene glycol diglycidyl ether represented by the following formula is preferred and includes more preferably diethylene glycol diglycidyl ether (n=2 in the following formula) and ethylene glycol diglycidyl ether (n=1 in the following formula).

[Formula 2]

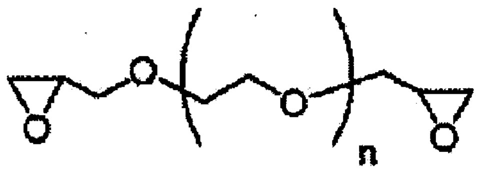

wherein n represents an integer in the range of 1 - 6, preferably in the range of 1 - 4.

In addition, the polyol, the polyamine and the polyisocyanate as the crosslinking agent includes preferably those having two functional groups such as diol, diamine and diisocyanate, more preferably $C_{3-8}$ alkane diol and $C_{3-8}$ alkane diisocyanate, and further preferably 1,6-hexan-diol or hexamethylene diisocyanate.

In the case where the carboxyl group of the glutamic acid as the building unit of the γ-polyglutamic acid is reacted with the hydroxyl group of the crosslinking agent, a condensation agent is preferably used and includes, for example, dimethylaminopyridine and dicyclohexylcarbodiimide.

In the present invention, commercially available crosslinking agents such as those provided from ALDRICH, TOKYO CHEMICAL INDUSTRY CO., LTD., and the like may be directly used or appropriately purified before use.

In the case where the polyepoxy compound is used as the crosslinking agent for preparing the chemically-crosslinked γ-polyglutamic acid, it may be prepared, for example, by the following method.

That is, a mixture of a non-crosslinked γ-polyglutamic acid and a basic compound such as sodium hydrogencarbonate, or a non-crosslinked γ-polyglutamate is dissolved in a solvent, and the polyepoxy compound was then added and stirred for reaction to give a clear and colorless chemically-crosslinked γ-polyglutamic acid.

In this connection, the temperature for adding and stirring steps can be appropriately set in the range of 0 - +100°C.

The solvent which can be used in the reaction is not particularly limited and includes, for example, water or dimethylsulfoxide, preferably water.

In the case where the diol is used as the crosslinking agent for preparing the chemically-crosslinked γ-polyglutamic acid, it may be prepared, for example, by the following method.

That is, the chemically-crosslinked γ-polyglutamic acid can be prepared by subjecting a non-crosslinked γ-polyglutamic acid and a diol to condensation reaction, optionally in the presence of a condensing agent such as dimethylaminopyridine and/or dicyclohexylcarbodiimide, at a temperature of about -60 - +80°C for 0.1 - 48 hours.

The solvent used in the reaction is preferably a high-polar solvent such as N,N-dimethylformamide, N,N-dimethylacetamide, dimethylsulfoxide or N-methylpyrrolidone, preferably N,N-dimethylformamide.

The chemically-crosslinked γ-polyglutamic acid prepared by the crosslinking reaction as described above can be used in the present invention after purification by removing the unreacted γ-polyglutamic acid, the crosslinking agent, the condensing agent, and the like by the standard technique, if necessary.

In the present invention, the chemically-crosslinked γ-polyglutamic acid is directly or optionally in admixture with other ingredients used as the organ-adhesion preventing agent. The organ-adhesion preventing agent according to the present invention is not particularly limited for the content of the chemically-crosslinked γ-polyglutamic acid as far as it contains the chemically-crosslinked γ-polyglutamic acid as the effective ingredient and exerts the organ-adhesion preventing effect, and can comprise the pharmaceutically acceptable additives such as a stabilizing agent, an anti-oxidant, a coloring agent, if necessary.

In the present invention, the chemically-crosslinked γ-polyglutamic acid is not particularly limited for the form thereof, and can be directly used, for example, in the form of gel, or it may be also used in the form of powder produced by lyophilization.

In addition, the organ-adhesion preventing agent according to the present invention is not particularly limited for the form thereof, comprises the chemically-crosslinked γ-polyglutamic acid, and may be in the form of, for example, powder, granule, solution, sol, gel (jelly), or sheet. According to the preferred embodiment of the present invention, the organ-adhesion preventing agent may be preferably in the form of solid to a certain extent, most preferably in the form of powder in light of its operability.

Furthermore, the adhesion-preventing agent according to the present invention can be also used as a mixture with the other ingredients effective for the prevention of adhesion, such as fibrin glue, sodium hyaluronate, a molecular target-based drug which targets a molecule relating to the formation of adhesion, and the like. It can be also used in combination by embedding it in the surface of the other adhesion-preventing agent sheet as well.

The adhesion-preventing agent according to the present invention is highly safe in that it prevents effectively the adhesion of the surfaces of local organs, as well as promotes the healing of an injury on the surface of the local organs and is absorbed in the body without side effects. Moreover, it may stop bleeding, which is of a small amount, and such hemostatic effect has not been found in the conventional adhesion-preventing agent.

According to another aspect of the present invention, a process for preventing the adhesion of organs is provided. The process for preventing the adhesion of organs according to the present invention comprises a step of bringing the adhesion-preventing agent into contact with or depositing the same onto the surface of a local organ and optionally the neighborhood thereof to be prevented from the post-operative adhesion. In this connection, the term "bringing into contact with or depositing onto" means herein the contact or depositting by coating or spraying the adhesion-preventing agent on the surface of a local organ and optionally the neighborhood thereof to be prevented from the adhesion.

The term "the surface of the local organ" means herein all or a part of the surface of an organ in which inflammation is caused by operations such as surgical operation or some etiology and thus it is required to prevent the adhesion.

The site or type of the organs is preferably, but not limited to, the organs other than the surface of the body (skin) of mammals including human being, experimental animal such as mouse, rat, hamster, rabbit, and the like, and domestic animal. The organs include, for example, digestive organs such as stomach, small intestine and large intestine, genital organs such as uterus and ovary, respiratory organs such as heart and lung, locomotoria such as muscle, bone, and ligament, sense organs such as eye, and the like.

The type of surgery of organs is not limited. The surface of the local organ may be a direct target of the surgery, or a damaged part which is not the direct target of the surgery but damaged as the result of the surgery.

The surface of the local organ is not limited to particular states. The adhesion-preventing agent of the present invention is water soluble and thus can be applied to the surface without being carried away even in wet state.

In the process for preventing adhesion of organs according to the present invention, the form of the adhesion-preventing agent and the percentage of the effective ingredient used for the step of bringing the adhesion-preventing agent into contact with or depositing the same onto the surface of the post-operative local organ and optionally the

neighborhood thereof to be prevented from the adhesion can be appropriately determined depending on the kinds of organs and the states of wound parts. As the adhesion-preventing agent according to the present invention causes no side effects and is highly safe, the amount of the adhesion-preventing agent contacted with the surface of the local organs can be set such that the surface is adequately covered.

The effect of preventing adhesion of organs according to the present invention, for example, in the case of the adhesion-preventing agent in the form of gel, is judged by visually observing the existence of adhesion after one week of coating the surface of local organs (for example, in wet state) with the adhesion-preventing agent. In general, the adhesion-preventing agent according to the present invention becomes invisible to the naked eye within about 48 hours after coating. In this connection, if the adhesion is not caused at this point of time, it can be judged that the adhesion is effectively prevented, although it depends on the types of the organs or the states of the surface of the organs.

## EXAMPLES

The present invention is described more specifically with reference to the following examples, but it is not limited to these

examples.

### Preparation Example 1

To a non-crosslinked γ-polyglutamic acid (referred to hereinafter as non-crosslinked PGA; Batch No.: 90630, Molecular Weight: 320,000 (measured by gel permeation chromatography-light scattering detection method (GPC-LS) with Pullulan as a standard) in an amount of 2 g (corresponding to 15.5 mmol as the total number of moles of glutamic acid as the monomer unit) were added 15.5 ml of pure water and 0.65 g of sodium hydrogen carbonate, and the mixture was completely dissolved. Diethylene glycol diglycidyl ether (n=2, Test Group Nos. 1 - 6) or ethylene glycol diglycidyl ether (n=1, Test Group Nos. 7 - 9) (both manufactured by ALDRICH) was added to this solution to perform reaction with stirring at 80°C for 1.5 hours. As the result, the reaction solution obtained exhibited the following characteristics. In this connection, the molecular number of the crosslinking agent : the total number of carboxyl groups contained in the γ-polyglutamic acid = the total number of moles of the crosslinking agent used : the total number of moles of glutamic acid which is the building unit of the γ-polyglutamic acid used.

[Table 1]

| Test Groups | Loads of crosslinking agent (mmol) | Ratio of total number of moles of crosslinking agent used and total number of moles of glutamic acid as the building unit of γ-polyglutamic acid used | n | Characteristics of reaction solution |
|---|---|---|---|---|
| 1 | 0.38 | 1:40 | 2 | Not gelled |
| 2 | 0.78 | 1:20 | | Gelled |
| 3 | 1.16 | 1:13.3 | | Gelled |
| 4 | 1.94 | 1:8 | | Gelled |
| 5 | 3.88 | 1:4 | | Gelled |
| 6 | 5.81 | 1:2.67 | | Gelled |
| 7 | 0.38 | 1:40 | 1 | Not gelled |
| 8 | 0.78 | 1:20 | | Gelled |
| 9 | 1.16 | 1:13.3 | | Gelled |

After reaction, 0.65 g of sodium hydrogen carbonate and appropriate amount of pure water were added to the gel obtained, which was swollen and then lyophilized at a reduced pressure of 0.08 Torr for 72 hours to give a chemically-crosslinked γ-polyglutamic acid (referred to hereinafter as PGA-CCC).

Preparation Example 2

To a solution of the non-crosslinked PGA (2 g, the same lot as in Preparation Example 1) and 1,6-hexan-diol (0.78 mmole, crosslinking agent) in N,N-dimethylformamide (DMF, 31 ml) were added dimethylaminopyridine (3.12 mmoles) and dicyclohexylcarbodiimide hydrochloride (3.12 mmoles), and the reaction was performed with stirring at 80°C for 1.5 hours. As the result, γ-polyglutamic acid was crosslinked to form a gel.
After reaction, the resulting gel was diluted and swollen with an appropriate amount of pure water, and then lyophilized at a reduced pressure of 0.08 Torr for 72 hours to give a PGA-CCC.

Preparation Example 3

To a solution of the non-crosslinked PGA (2 g, the same lot as in Preparation Example 1) were added pure water (15.5 ml) and sodium hydrogen carbonate (0.65 g), and the mixture was completely dissolved. To this solution was added ethylene glycol diglycidyl ether (purified by distillation of the commercially available product of ALDRICH, density: 1.12 g/$cm^3$), and the mixture was reacted with stirring at 80°C for 2 hours.
After reaction, the resulting gel was mixed and swollen with sodium hydrogen carbonate (0.325 g) and an appropriate amount of pure water, and then lyophilized at a reduced pressure of 0.08 Torr for 72 hours to give a PGA-CCC.
As the result, the reaction solution obtained exhibited the following characteristics, and the gels were viscous in the test groups 1 and 2, fluid in the test group 3 and thin in the test groups 4 - 7.
About 0.1 g of each sample prepared in the test group was precisely weighed and placed in a one-liter beaker, and 1 liter of deionized water was added thereto. The top of the beaker was covered with aluminum foil and left standing overnight in a low temperature chamber at 5°C in order to avoid the influence of temperature. A two-liter beaker was covered with four layers of gauzes (Japanese Pharmacopoeia; type I), onto which the content of the one-liter beaker was poured. After pouring all of the content, gel, if remained in the one-liter beaker, was scraped off with a spatula and placed on the gauzes. After being left standing for 1 hour, the gel remained on the gauzes were placed in a 500 ml beaker of which tare weight had been preliminarily measured. The gel sticked to the gauzes was also scraped off with a spatula to place into the 500 ml beaker. The tare weight of the 500 ml beaker was subtracted from the total weight of the beaker at this time to obtain the value as the water absorption. In addition, the water absorption coefficient (g/g) was calculated from the following equation. The results are shown in Table 2 below, and the water absorption coefficients of chemically-crosslinked γ-polyglutamic acid were high in the range of several hundreds.

[Equation 1]

$$\text{Water absorption coefficient (g/g)} = \text{Water absorption (g)/Sample weights (g)}$$

[Table 2]

| Test Group | Loads of crosslinking agent (mmol) | Ratio of total number of moles of crosslinking agent used and total number of moles of glutamic acid as the building unit of γ-polyglutamic acid used | Characteristics of reaction solution | Water absorption coefficient |
|---|---|---|---|---|
| 1 | 1.09 | 1:14 | Viscous gel | 230 - 540 |
| 2 | 0.96 | 1:16 | Viscous gel | 600 - 690 |
| 3 | 0.77 | 1:20 | Fluid gel | 610 - 710 |
| 4 | 0.64 | 1:24 | Thin gel | 340 - 390 |
| 5 | 0.45 | 1:34 | Thin gel | |

(continued)

| Test Group | Loads of crosslinking agent (mmol) | Ratio of total number of moles of crosslinking agent used and total number of moles of glutamic acid as the building unit of γ-polyglutamic acid used | Characteristics of reaction solution | Water absorption coefficient |
|---|---|---|---|---|
| 6 | 0.32 | 1:48 | Thin gel | |

Example 1: Organ-adhesion preventing effect

In order to confirm the effect of PGA-CCC on preventing organs from adhesion, an organ treated with the PGA-CCC of the test group 2 prepared in Preparation Example 1 was compared with the control untreated with PGA-CCC.

Two Donryu rats (male, 5 - 7 weeks old) were used for the experiment. The rats were subjected to laparotomy under general anesthetization (abdominal midline incision) to excise the abdominal wall right above the ileocecum of large intestine in a size of about 1 x 2 cm. The ileocecum was then moved out of the abdominal cavity, and the surface of the serous membrane of the ileocecum was rubbed with gauze until the appearance of small bleeding spots. Furthermore, the surface was heated with a drier for about 20 seconds and then dried in the room air for about 10 minutes.

0.2g of the PGA-CCC was coated on the part rubbed in the ileocecum and the part where peritoneum and abdominal wall had been excised.

The degree of adhesion in the test group 2 of the PGA-CCC of the above Table 1 was observed by laparotomy after about a week. As a result, no adhesion was observed in two animals, which reveals that the PGA-CCC is effective for preventing the adhesion of organs. In addition, the state of the non-treated group upon laparotomy after a week is shown in Fig. 1, and the state of the effect of the PGA-CCC treatment for preventing the adhesion of organs is shown in Fig. 2.

Example 2: Organ-adhesion preventing effect

Organ-adhesion preventing effect was confirmed with use of the PGA-CCCs of the test groups 1 - 5 in the similar manner to Example 1.

After about a week, the degree of adhesion in each group was observed by laparotomy, and the adhesion was evaluated on the basis of grades consisting of "none" where no adhesion was observed, "low" where blunt dissection can be done, "moderate" where sharp dissection can be done, and "high" where adhesion was observed in such an extent that the organs are damaged by dissection. As shown in Table 3 below, high degree of adhesion was observed in all of the individuals in the non-treated group, while no adhesion was observed in any of five animals of the test groups 1 - 5, which reveals that the PGA-CCCs are effective for preventing the adhesion of organs.

[Table 3]

| Extent of adhesion / Sample | None | Low | Moderate | High | Total |
|---|---|---|---|---|---|
| Test group 1 | 5 | 0 | 0 | 0 | 5 |
| Test group 2 | 5 | 0 | 0 | 0 | 5 |
| Test group 3 | 5 | 0 | 0 | 0 | 5 |
| Test group 4 | 5 | 0 | 0 | 0 | 5 |
| Test group 5 | 5 | 0 | 0 | 0 | 5 |
| Non-treated group | 0 | 0 | 0 | 5 | 5 |

**Claims**

1. An organ-adhesion preventing agent comprising a chemically-crosslinked γ-polyglutamic acid as an effective ingredient.

2. The organ-adhesion preventing agent according to Claim 1, wherein the chemically-crosslinked γ-polyglutamic acid is produced by using a crosslinking agent and a γ-polyglutamic acid in a ratio in the range of 1:100 - 1:2, the ratio being of the total number of moles of the crosslinking agent and the total number of moles of the glutamic acid as the building unit of the γ-polyglutamic acid.

3. The organ-adhesion preventing agent according to Claim 2, wherein said ratio is in the range of 1:40 - 3:8.

4. The organ-adhesion preventing agent according to Claim 2, wherein said ratio is in the range of 1:35 - 1:10.

5. The organ-adhesion preventing agent according to any one of Claims 1 to 4, wherein said chemically-crosslinked γ-polyglutamic acid is chemically crosslinked by a crosslinking agent selected from the group consisting of polyepoxy compound, polyol, polyamine, polyisocyanate, diol, diamine and diisocyanate.

6. The organ-adhesion preventing agent according to Claim 5, wherein said polyepoxy compound is (poly)ethylene glycol diglycidyl ether, (poly)propylene glycol diglycidyl ether, $C_{3-8}$ alkane diol or $C_{3-8}$ alkane diisocyanate.

7. The organ-adhesion preventing agent according to Claim 6, wherein said crosslinking agent is diethylene glycol diglycidyl ether, ethylene glycol diglycidyl ether, 1,6-hexan-diol or hexamethylene diisocyanate.

8. The organ-adhesion preventing agent according to any one of Claims 1 to 7, wherein said chemically-crosslinked γ-polyglutamic acid is obtained by reacting γ-polyglutamic acid or a salt thereof with a crosslinking agent in a solvent.

9. The organ-adhesion preventing agent according to Claim 1, wherein said organ-adhesion preventing agent is in the form of powder, granule, solution, sol, gel or sheet.

10. A process for preventing organ from adhesion, comprising a step of: bringing the adhesion-preventing agent according to Claim 1 into contact with or depositing the same onto the surface of a local organ and optionally the neighborhood thereof to be prevented from the post-operative adhesion.

11. The process for preventing organ from adhesion according to Claim 10, wherein the surface of the local organ is in wet state.

**12.** A use of a chemically-crosslinked γ-polyglutamic acid as an organ-adhesion preventing agent.

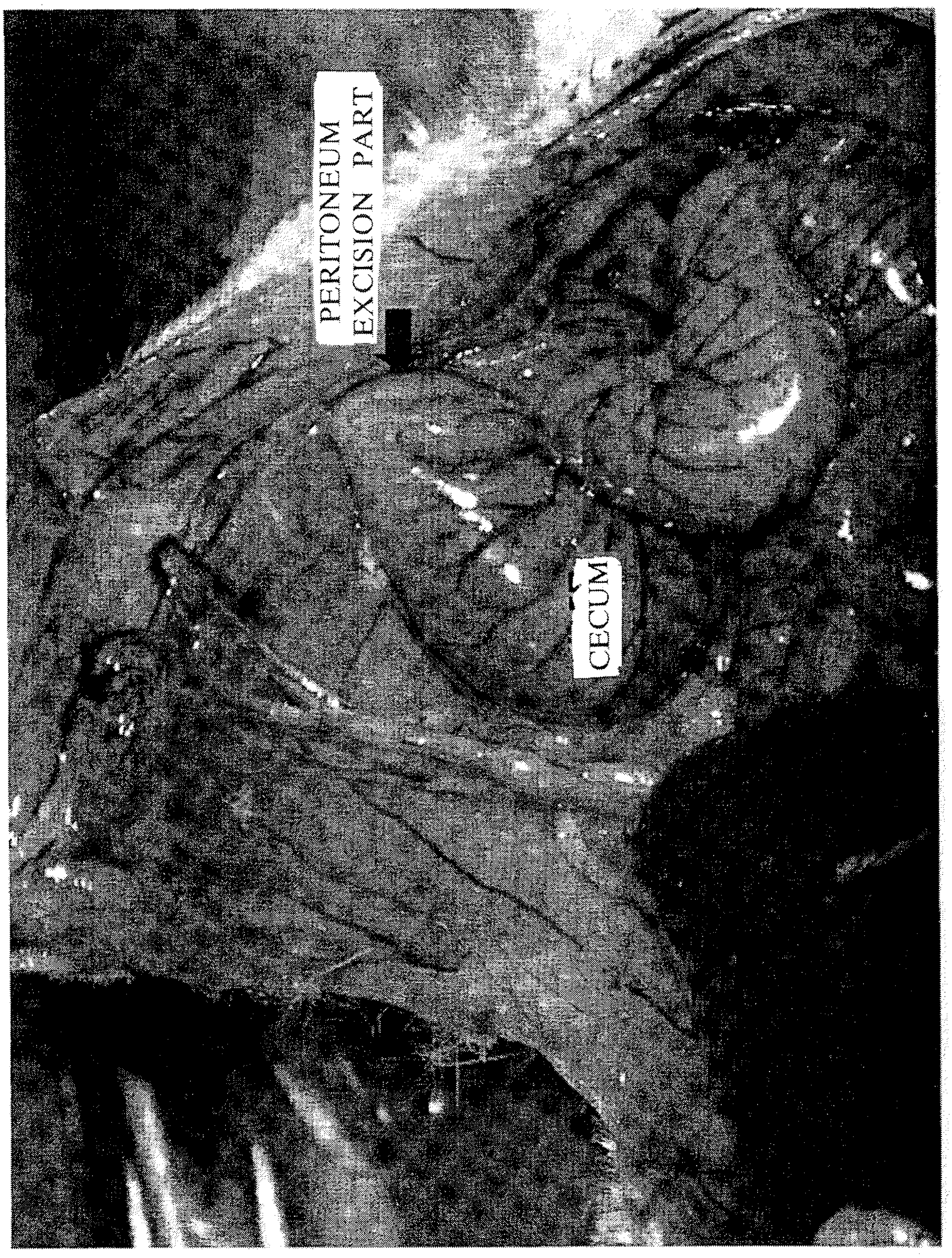


FIG. 1

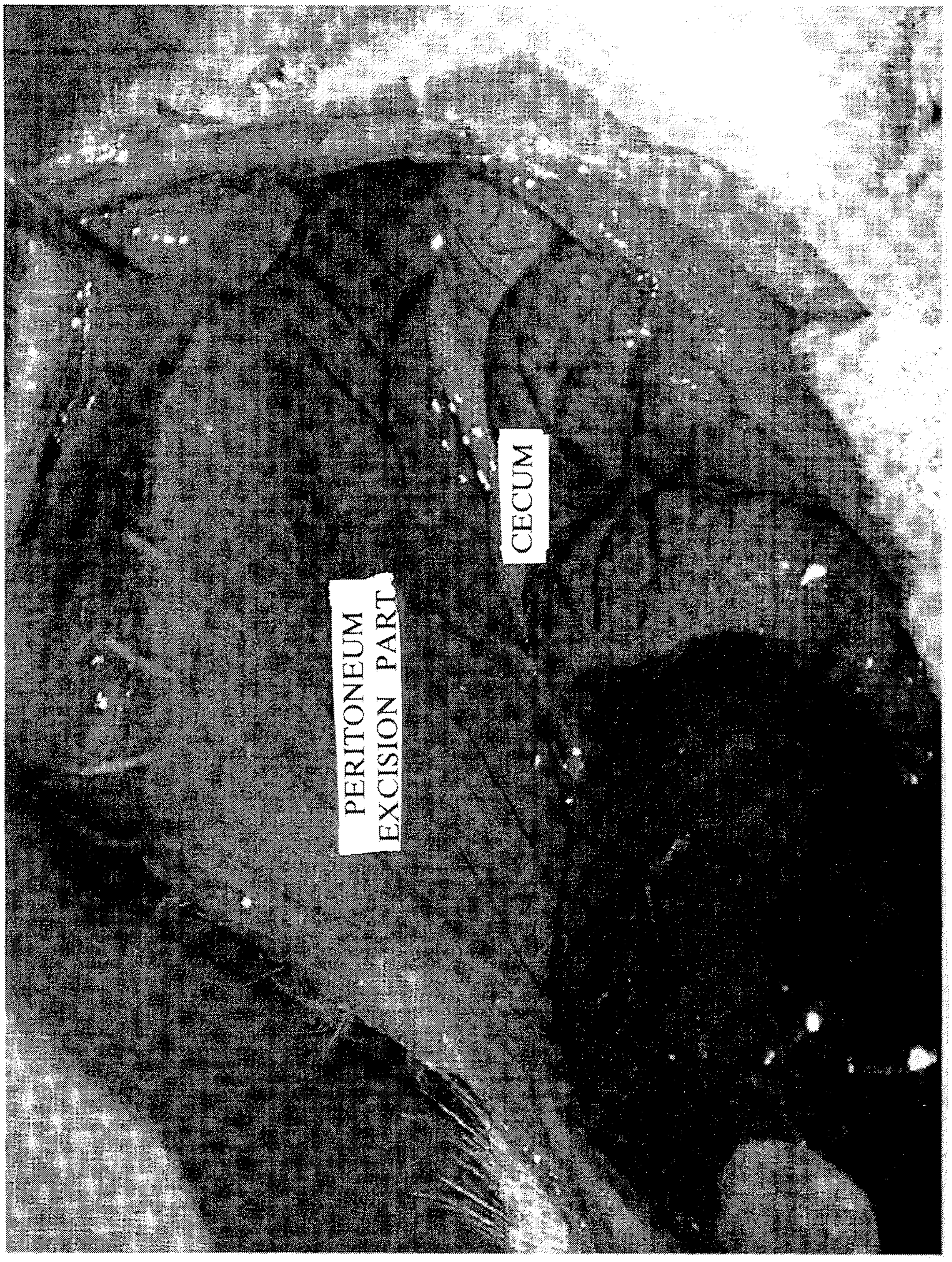


FIG. 2

## INTERNATIONAL SEARCH REPORT

International application No.
PCT/JP2007/059770

A. CLASSIFICATION OF SUBJECT MATTER
*A61L31/00*(2006.01)i, *A61K31/785*(2006.01)i, *A61P7/04*(2006.01)i, *A61P41/00*(2006.01)i

According to International Patent Classification (IPC) or to both national classification and IPC

B. FIELDS SEARCHED

Minimum documentation searched (classification system followed by classification symbols)
A61L31/00, A61K31/785, A61P7/04, A61P41/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 10-155892 A (Kuraray Co., Ltd.),<br>16 June, 1998 (16.06.98),<br>Par. Nos. [0016] to [0018], [0049], [0101]<br>& EP 838224 A2 & US 5980883 A | 1,5,8,9<br>2-4,6,7 |
| X<br>Y | JP 2006-95097 A (Terumo Corp.),<br>13 April, 2006 (13.04.06),<br>Claims; Par. Nos. [0052], [0054]<br>(Family: none) | 1,8,9<br>2-7 |
| Y | JP 2002-145990 A (Mitsui Chemicals, Inc.),<br>22 May, 2002 (22.05.02),<br>Par. Nos. [0095], [0100] to [0101]; example 5<br>(Family: none) | 2-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
06 June, 2007 (06.06.07)

Date of mailing of the international search report
19 June, 2007 (19.06.07)

Name and mailing address of the ISA/
Japanese Patent Office

Facsimile No.

Authorized officer

Telephone No.

Form PCT/ISA/210 (second sheet) (April 2005)

**INTERNATIONAL SEARCH REPORT**

International application No.
PCT/JP2007/059770

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2005-145908 A (Nishikawa Rubber Co., Ltd.), 09 June, 2005 (09.06.05), Claims; example 2 (Family: none) | 2-4 |
| Y | JP 7-310021 A (Nippon Shokubai Co., Ltd.), 28 November, 1995 (28.11.95), Par. No. [0049] & EP 668080 A2 & US 5610208 A & US 2002/0013394 A1 & US 2003/0092849 A1 | 5-7 |
| P,X | WO 2006/054624 A1 (Keio University), 26 May, 2006 (26.05.06), Full text (Family: none) | 1-9 |

Form PCT/ISA/210 (continuation of second sheet) (April 2005)

**INTERNATIONAL SEARCH REPORT**

International application No.
PCT/JP2007/059770

**Box No. II Observations where certain claims were found unsearchable (Continuation of item 2 of first sheet)**

This international search report has not been established in respect of certain claims under Article 17(2)(a) for the following reasons:

1. [x] Claims Nos.: 10-12
because they relate to subject matter not required to be searched by this Authority, namely:
Claims 10 to 12 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006136991 A
- JP 8266615 A
- JP 11076572 A
- JP 2003000695 A
- JP 6322358 A
- JP 6256220 A
- JP 4298533 A
- JP 11343339 A
- JP 2002128899 A
- JP 2005021094 W
- JP 1174397 A
- JP 7316286 A

### Non-patent literature cited in the description

- *Biosci. Biotech.,* 1992, vol. 56, 1031-1035